# FASCICULE DE BREVET EUROPEEN

(11) **EP 0 462 017 B1**
(45) Date de publication et mention de la délivrance du brevet: **28.07.1993**
(21) Numéro de dépôt: 91401583.9
(22) Date de dépôt: 14.06.1991
(51) Int. Cl.: C07D 209/08, A61K 31/40

(54) **Procédé de préparation industrielle du 3-sulfamoyl 4-chloro N-(2,3-dihydro 2-méthyl 1H-indol-1-yl) benzamide à partir du 2,3-dihydro 2-méthyl 1H-indole et de l'acide hydroxylamine-O-sulfonique**
Verfahren zur industriellen Herstellung von 3-Sulfamoyl-4-chlor-N-(2,3-dihydro-2-methyl-1H-indol-1-yl)-Benzamide ausgehend von 2,3-Dihydro-2-methyl-1H-indol und Hydroxylamine-O-sulfonsäure
Process for the industrial preparation of 3-sulfamoyl-4-chloro-N-(2,3-dihydro-2-methyl-1H-indol-1-yl)benzamide from 2,3-dihydro-2-methyl-1H-indole and hydroxylamine-O-sulfonic acid

(30) Priorité: 14.06.1990 FR 9007387
(43) Date de publication de la demande: 18.12.1991
(73) Titulaire: ADIR ET COMPAGNIE, 92415 Courbevoie Cédex (FR)
(72) Inventeur: Thevignot, Roger, F-76210 Bolbec (FR)
(74) Mandataire: Reverbori, Marcelle

(56) Documents cités:
- EP-A- 0 054 892
- EP-A- 0 068 239
- FR-A- 2 003 311
- US-A- 4 564 677

## Description

La présente invention concerne un nouveau procédé de préparation industrielle du 4-chloro 3-sulfamoyl N-(2,3-dihydro 2-méthyl 1H-indol-1-yl) benzamide.

Ce composé aussi connu sous la DCI indapamide, possède des propriétés pharmacologiques très intéressantes. Il est doué notamment de propriétés antihypertensives et il est utilisé pour le traitement de l'hypertension artérielle essentielle.

Plusieurs méthodes de préparation de ce composé sont déjà connues. Toutefois, les procédés déjà décrits dans la littérature, ne permettent pas toujours d'obtenir l'indapamide avec une pureté satisfaisante ou avec un bon rendement. De plus, certaines étapes de ces procédés posent des problèmes au niveau industriel.

Le procédé de préparation du 4-chloro 3-sulfamoyl N-(2,3-dihydro 2-méthyl 1H-indol-1-yl) benzamide décrit dans le brevet FR 2.003.311 consiste à obtenir ce composé en faisant réagir le chlorure de 4-chloro 3-sulfamoyl benzoyle sur la 1-amino 2,3-dihydro 2-méthyl indole. Ce dernier composé est préparé selon le procédé de J.B. Wright et R.E. Willette (J. Med. and Pharm. Chem. 5 811 (1962) qui comprend la réduction de l'analogue N-nitrosé. Le rendement des différentes étapes de ce procédé est peu satisfaisant. Par ailleurs, ce procédé de synthèse conduit à l'obtention de produits secondaires, notamment des dérivés de nitrosamines, qui sont des composés de toxicité potentielle.

Le brevet JP 54.030.159 décrit un procédé de préparation de l'indapamide à partir de 4-chloro 3-sulfamoyl N-(2-méthyl indol-1-yl) benzamide, qui est transformé en indapamide après réduction. Ce procédé conduit aussi à la formation d'un grand nombre de produits secondaires, et plusieurs purifications sont nécessaires pour obtenir un produit de qualité pharmaceutique.

Un autre procédé de préparation de l'indapamide est décrit dans le brevet EP 54.892. Ce procédé consiste à cycliser la 1-allyl 1-phényl 2-(3-sulfamoyl 4-chlorobenzoyl) hydrazine en présence d'acides de Lewis ou de Bronsted. Cette cyclisation conduit aussi à la formation d'un grand nombre de produits secondaires, et le rendement de ce procédé est peu satisfaisant.

Compte-tenu de l'intérêt thérapeutique de l'indapamide, et l'absence d'un procédé industriel permettant son obtention avec une pureté satisfaisante, un bon rendement et si possible, à partir de matières premières pas très onéreuses et disponibles dans le commerce, des recherches plus approfondies ont été entreprises et ont abouti à la découverte d'un nouveau procédé de préparation du 4-chloro 3-sulfamoyl N-(2,3-dihydro 2-méthyl 1H-indol-1-yl) benzamide. Le stade final de ce procédé est proche de celui décrit dans le brevet FR 2.003.311 mais il présente deux grands avantages : il nécessite moins d'étapes, et la préparation de la 1-amino 2,3-dihydro 2-méthyl 1H-indole est réalisée sans formation de l'analogue N-nitrosé. L'application industrielle de ce procédé est donc très avantageuse.

La présente invention a plus particulièrement pour objet un procédé de synthèse industrielle du 4-chloro 3-sulfamoyl N-(2,3-dihydro 2-méthyl 1H-indol-1-yl) benzamide, composé de formule I : 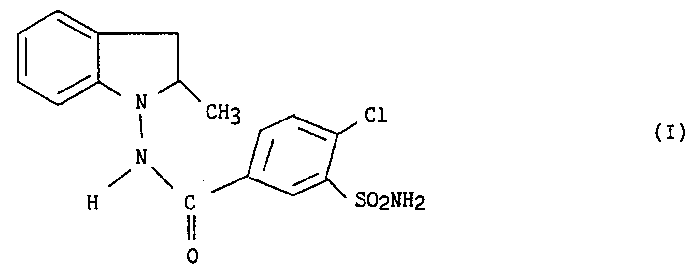
caractérisé en ce que l'on soumet le 2,3-dihydro 2-méthyl 1H-indole, composé de formule II : 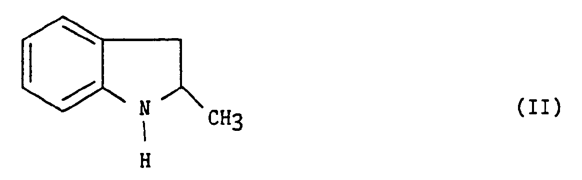
à l'action de l'acide hydroxylamine-O-sulfonique, composé de formule III :

H₂NOSO₃H (III)

en présence de triéthylamine, et à une température comprise entre 20°C et 60°C,
puis l'on sépare du milieu réactionnel par extraction sélective le 1-amino 2,3-dihydro 2-méthyl 1H-indole, composé de formule IV : 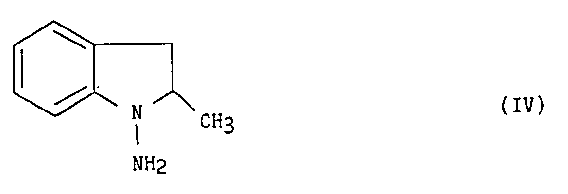
que l'on fait réagir, sous forme de base ou sous forme de sel, en solution dans le tétrahydrofurane ou en milieu alcoolique et en présence d'un accepteur d'acide avec le chlorure de 4-chloro 3-sulfamoyl benzoyle, composé de formule V : 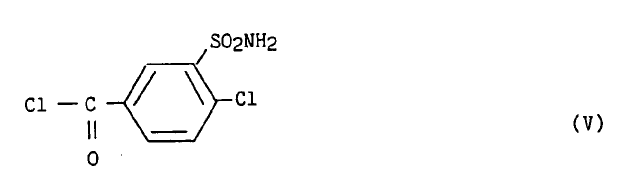
à une température comprise entre 20° et 30°C pour former le composé de formule I.

Lors de la préparation de la 1-amino 2,3-dihydro 2-méthyl 1H-indole, le rapport de la quantité molaire mis en réaction de l'acide hydroxylamine-o-sulfonique sur la quantité molaire du 2,3-dihydro 2-méthyl 1H-indole doit être compris entre 1 et 5, et de manière préférentielle entre 1,5 et 2,5.

La réaction est réalisée en présence d'une quantité équimolaire de triéthylamine par rapport à l'acide hydroxylamine-O-sulfonique (1,5 mole pour 1 mole de 2,3-dihydro 2-méthyl 1H-indole). Comme solvant on peut utiliser le chlorure de méthylène, le dichloroéthane, le chloroforme ou d'autres alcanes polyhalogénés de petit poids moléculaire (C₁ - C₅). La durée de la réaction dépend de la température à laquelle la réaction est réalisée.
A 24°C, pour obtenir un rendement satisfaisant, la durée de la réaction doit être d'environ 24 heures tandis qu'à 50°C, 4 heures sont nécessaires.

L'acide hydroxylamine-O-sulfonique est un produit commercial (Aldrich ®, Janssen ®).

A la fin de la réaction, après alcalinisation du milieu réactionnel à l'aide d'une base minérale forte, le 1-amino 2,3-dihydro 2-méthyl 1H-indole est isolé dans la phase organique (décantation et contre-extraction).

Le 1-amino 2,3-dihydro 2-méthyl 1H-indole, ainsi obtenu, peut-être salifié avant d'être utilisé pour la préparation de l'indapamide. De manière préférentielle, on peut utiliser pour la salification, l'acide chlorhydrique ou l'acide méthanesulfonique.

La réaction du 1-amino 2,3-dihydro 2-méthyl 1H-indole avec le chlorure de 4-chloro 3-sulfamoyl benzoyle est effectuée en présence d'un excès de triéthylamine. Comme solvant réactionnel, on peut utiliser le tétrahydrofurane ou un autre solvant chimiquement équivalent.

Ci-après est donnée à titre non limitatif une description détaillée de la mise en oeuvre du procédé de l'invention.

### EXEMPLE 1

### Préparation du 1-amino 2,3-dihydro 2-méthyl 1H-indole et de son chlorhydrate

Dans un réacteur purgé à l'azote et sous courant d'azote, charger 22,27 g d'acide hydroxylamine-O-sulfonique en suspension dans 22 ml de chlorure de méthylène.
Couler ensuite en 1 heure à 24°C, 17,5 g de 2,3-dihydro 2-méthyl 1H-indole et 19,98 g de triéthylamine en solution dans 10 ml de chlorure de méthylène.

Laisser la réaction se poursuivre pendant 24 heures. Ensuite, ajouter tout en refroidissant 100 ml d'un mélange d'eau et d'ammoniaque concentrée (80 : 20 V/V). Agiter pendant 15 mn, puis décanter, récupérer la phase organique et réextraire la phase aqueuse avec 20 ml de chlorure de méthylène.
Réunir les phases organiques et les laver avec 30 ml d'eau.

Eliminer sous vide au rotavapor le chlorure de méthylène pour obtenir le 1-amino 2,3-dihydro 2-méthyl 1H-indole.
- Rendement : 78 %
- Point de fusion : 37 - 38°C

Reprendre le résidu dans 140 ml de toluène, sous azote et en refroidissant, additionner goutte à goutte 14 ml d'acide chlorhydrique 12N. Agiter 2 heures à 20-22°C, puis filtrer et laver avec du toluène.

Réempâter mécaniquement pendant 30 mn par 50 ml d'un mélange de toluène et d'acétonitrile (15 : 45 V/V). Filtrer et sécher à l'étuve.
Pureté du chlorhydrate du 1-amino 2,3-dihydro 2-méthyl 1H-indole :
99,3 % (C.G.L.)

### EXEMPLE 2

### Préparation du 4-chloro 3-aminosulfonyl N-(2,3-dihydro 2-méthyl 1H-indol-1-yl) benzamide

Dans un réacteur, purgé à l'azote, on charge 800 ml de tétrahydrofurane et 0,2045 mole de chlorhydrate du 1-amino 2,3-dihydro 2-méthyl 1H-indole. On coule ensuite en 10 minutes 0,4328 mole de triéthylamine. La solution réactionnelle est agitée ensuite à 18 - 20°C pendant 30 minutes. On coule alors goutte à goutte en 1 h 45 mn 0,2050 mole de chlorure de 4-chloro 3-sulfamoyl benzoyle en solution dans 400 ml de tétrahydrofurane.

On laisse le milieu réactionnel sous agitation pendant 3 heures à 24 - 30°C, puis on ajoute 0,5 g de Noir CN₁®. On filtre et on concentre le filtrat.

Le résidu de l'évaporation est ensuite recristallisé dans l'isopropanol.
- Rendement : 80,5 %
- Pureté (CLHP) : 99,98 %

## Revendications

1. Procédé de préparation du 4-chloro 3-sulfamoyl N-(2,3-dihydro 2-méthyl 1H-indol-1yl) benzamide, composé de formule I : 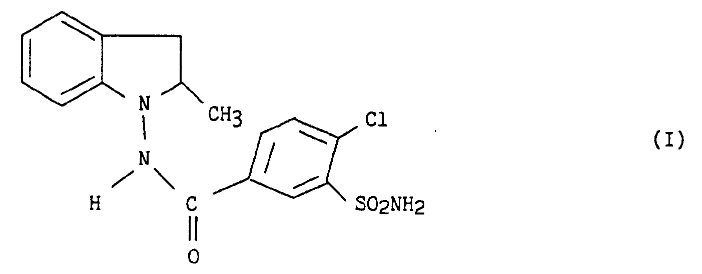 caractérisé en ce que l'on soumet le 2,3-dihydro 2-méthyl 1H-indole, composé de formule II : 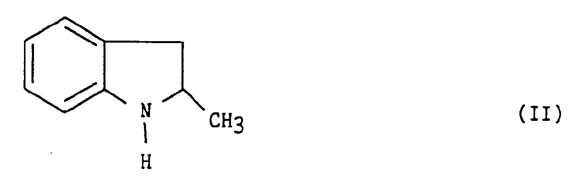 à l'action de l'acide hydroxylamine-O-sulfonique, composé de formule III :
H₂NOSO₃H (III)
en suspension dans un alcane polyhalogéné, en présence de triéthylamine, et à une température comprise entre 20°C et 60°C,
puis que l'on sépare du milieu réactionnel par extraction sélective le 1-amino 2,3-dihydro 2-méthyl 1H-indole, composé de formule IV : 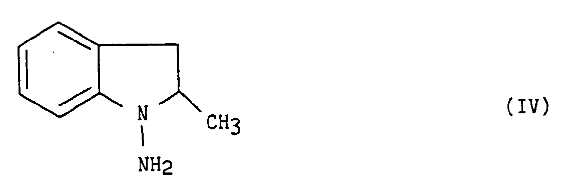 que l'on fait réagir, sous forme de base ou sous forme de sel, en solution dans le tétrahydrofurane ou en milieu alcoolique et en présence d'un accepteur d'acide avec le chlorure de 4-chloro 3-sulfamoyl benzoyle, composé de formule V : 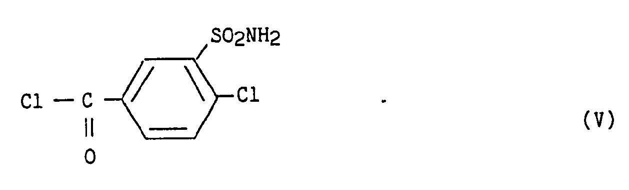 à une température comprise entre 20° et 30°C pour former le composé de formule I.

2. Procédé selon la revendication 1 caractérisé en ce que le 2,3-dihydro 2-méthyl 1H-indole est en solution dans le chlorure de méthylène ou dans le dichloroéthane

3. Procédé selon la revendication 1 caractérisé en ce que le rapport de la quantité molaire mis en réaction de l'acide hydroxylamine-O-sulfonique sur la quantité molaire du 2,3-dihydro 2-méthyl 1H-indole est compris entre 1 et 5.

4. Procédé selon la revendication 1 caractérisé en ce que la réaction de l'acide hydroxylamine-O-sulfonique avec le 2,3-dihydro 2-méthyl 1H-indole est réalisée à une température comprise entre 20 et 60°C.

5. Procédé selon la revendication 1 caractérisé en ce que l'on fait réagir le 1-amino 2,3-dihydro 2-méthyl 1H-indole sous forme de chlorhydrate ou de mésylate, avec le chlorure de 4-chloro 3-sulfamoyl benzoyle.

## Patentansprüche

1. Verfahren zur Herstellung von 4-Chlor-3-sulfamoyl-N-(2,3-dihydro-2-methyl-1H-indoly-1-yl)-benzamid der Formel I 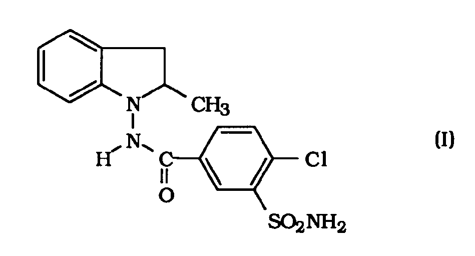 **dadurch gekennzeichnet**, daß man 2,3-Dihydro-2-methyl-1H-indol der Formel II 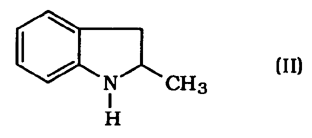 der Einwirkung von Hydroxylamin-O-sulfonsäure der Formel III
H₂NOSO₃H (III)
in Suspension in einem polyhalogenierten Alkan in Gegenwart von Triethylamin und bei einer Temperatur zwischen 20°C und 60°C unterwirft, durch selektive Extraktion 1-Amino-2,3-dihydro-2-methyl-1H-indol der Formel IV 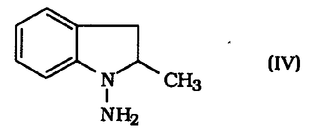 aus dem Reaktionsmedium abtrennt und in Form der Base oder in Form des Salzes in Lösung in Tetrahydrofuran oder in einem alkoholischen Medium in Gegenwart eines Säureakzeptors mit 4-Chlor-3-sulfamoyl-benzoylchlorid der Formel V 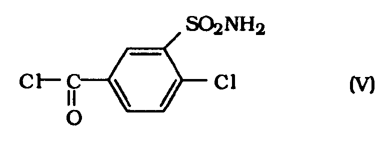 bei einer Temperatur zwischen 20 und 30°C umsetzt zur Bildung der Verbindung der Formel I.

2. Verfahren nach Anspruch 1, **dadurch gekennzeichnet**, daß man 2,3-Dihydro-2-methyl-1H-indol in Lösung in Methylenchlorid oder in Dichlorethan einsetzt.

3. Verfahren nach Anspruch 1, **dadurch gekennzeichnet**, daß das Verhältnis der Molmenge der bei der Reaktion eingesetzten Hydroxylamin-O-sulfonsäure zu der Molmenge von 2,3-Dihydro-2-methyl-1H-indol zwischen 1 und 5 liegt.

4. Verfahren nach Anspruch 1, **dadurch gekennzeichnet**, daß die Umsetzung von Hydroxylamin-O-sulfonsäure mit 2, 3-Dihydro-2-methyl-1H-indol bei einer Temperatur zwischen 20 und 60°C durchgefahrt wird.

5. Verfahren nach Anspruch 1, **dadurch gekennzeichnet,** daß man 1-Amino-2,3-dihydro-2-methyl-1H-indol in Form des Hydrochlorids oder des Mesylats mit 4-Chlor-3-sulfamoyl-benzoylchlorid umsetzt.

## Claims

1. Process for the preparation of 4-chloro-3-sulphamoyl-N-(2,3-dihydro-2-methyl-1H-indol-1-yl)benzamide, a compound of the formula I: 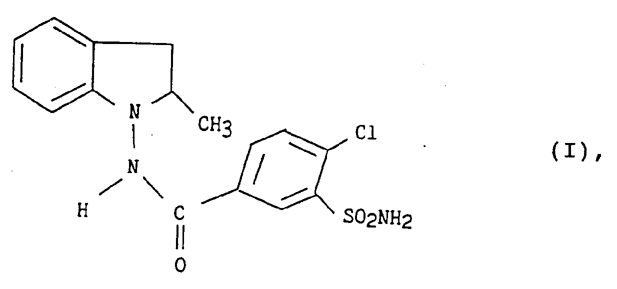 characterised in that 2,3-dihydro-2-methyl-1H-indole, a compound of the formula II: 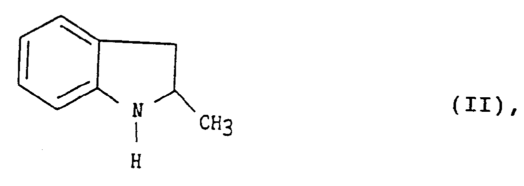 is subjected to the action of hydroxylamine-O-sulphonic acid, a compound of the formula III:
H₂NOSO₃H (III)
in suspension in a polyhalogenated alkane, in the presence of triethylamine and at a temperature of from 20°C to 60°C,
then 1-amino-2,3-dihydro-2-methyl-1H-indole, a compound of the formula IV: 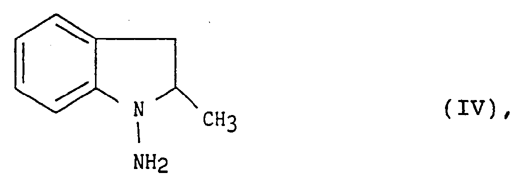 is separated from the reaction medium by selective extraction and is reacted in the form of a base or in the form of a salt, in solution in tetrahydrofuran or in alcoholic medium and in the presence of an acid acceptor, with 4-chloro-3-sulphamoylbenzoyl chloride, a compound of the formula V: 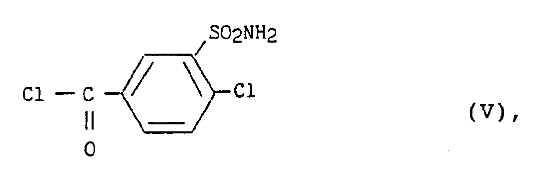 at a temperature of from 20° to 30°C, to form the compound of the formula I.

2. Process according to claim 1, characterised in that the 2,3-dihydro-2-methyl-1H-indole is in solution in methylene chloride or in dichloroethane.

3. Process according to claim 1, characterised in that the ratio of the molar amount of hydroxylamine-O-sulphonic acid to the molar amount of 2,3-dihydro-2-methyl-1H-indole reacted is from 1 to 5.

4. Process according to claim 1, characterised in that the reaction of hydroxylamine-O-sulphonic acid with 2,3-dihydro-2-methyl-1H-indole is carried out at a temperature of from 20 to 60°C.

5. Process according to claim 1, characterised in that the 1-amino-2,3-dihydro-2-methyl-1H-indole is reacted in the form of the hydrochloride or the methanesulphonate with the 4-chloro-3-sulphamoylbenzoyl chloride.
